# EUROPEAN PATENT APPLICATION

(11) **EP 2 634 256 A1**
(43) Date of publication of application: **04.09.2013**
(21) Application number: 12305248.2
(22) Date of filing: 01.03.2012
(51) Int. Cl.: C12N 15/74, C12N 15/90

(54) **De novo integron recombination sites and uses thereof**

(71) Applicant: Institut Pasteur, 75015 Paris (FR); Centre National de la Recherche Scientifique, 75016 Paris (FR)
(72) Inventor: Mazel, Didier, 92150 Suresnes (FR); Bikard, David, New York, NY 10028 (US); Grieb, Maj Svea, 01069 Dresden (DE)
(74) Representative: Thomas, Dean

(57) **Abstract**

The present invention relates to the fields of molecular biology and biotechnology and to *de novo* attC integron recombination sites and the use of these. In particular the present invention relates to isolated polynucleotides encoding synthetic attC sites comprising the sequence SEQ ID NO: 1, well as vectors comprising such polynucleotides. The present invention also relates to the use of such materials and methods to generate and test synthetic attC sites.

SEQUENCE LISTING

<110> Institut Pasteur
Centre National de la Recherche Scientifique

<120> De novo integron recombination sites and uses thereof

<130> F226 - 191 EP

<160> 26

<170> PatentIn version 3.4

<210> 1
<211> 10
<212> DNA
<213> Artificial

<220>
<223> Consensus for attCr site

<220>
<221> misc_feature
<222> (1)..(1)
<223> comprises at least 4 nucleotides of which the third from last
nucleotide is A and is complementary to N9

<220>
<221> misc_feature
<222> (2)..(2)
<223> if present comprises no more than 2 nucleotides

<220>
<221> misc_feature
<222> (3)..(3)
<223> comprises 6 to 10 nucleotides and is non-complimentary to N7

<220>
<221> misc_feature
<222> (4)..(4)
<223> comprises 9 to 30 nucleotides and is complimentary to N6

<220>
<221> misc_feature
<222> (5)..(5)
<223> comprises 3 to 50 nucleotides

<220>
<221> misc_feature
<222> (6)..(6)
<223> comprises 9 to 30 nucleotides and is complimentary to N4 which
comprises at least one nucleotide which forms a extra helical
base pair when N4 and N6 form a double helical strand

<220>
<221> misc_feature
<222> (7)..(7)
<223> comprises 6 to 10 nucleotides and is non-complimentary to N3

<220>
<221> misc_feature
<222> (8)..(8)
<223> if present comprises no more than 2 nucleotides

<220>
<221>  misc_feature
<222>  (9)..(9)
<223>  comprises at least 4 nucleotides of which the second from last
      nucleotide is T and is complementary to N1

<220>
<221>  misc_feature
<222>  (10)..(10)
<223>  any nucleotide if present

<400>  1
nnnnnnnnnn                                                              10


<210>  2
<211>  13
<212>  DNA
<213>  Artificial

<220>
<223>  attCr specific consensus



<220>
<221>  misc_feature
<222>  (5)..(5)
<223>  comprises 6 to 10 nucleotides and is non-complimentary to N9

<220>
<221>  misc_feature
<222>  (6)..(6)
<223>  comprises 9 to 30 nucleotides and is complimentary to N8

<220>
<221>  misc_feature
<222>  (7)..(7)
<223>  comprises 3 to 50 nucleotides

<220>
<221>  misc_feature
<222>  (8)..(8)
<223>  comprises 9 to 30 nucleotides and is complimentary to N6 which
      comprises at least one nucleotide which forms a extra helical
      base pair when N6 and N8 form a double helical strand

<220>
<221>  misc_feature
<222>  (9)..(9)
<223>  comprises 6 to 10 nucleotides and is non-complimentary to N5

<400>  2
taacnnnnng tta                                                          13


<210>  3
<211>  67
<212>  DNA
<213>  Artificial

<220>
<223>  r0-top

<400>  3
aattcattat aacggaggtt acccatggat tcgagttcct cgaaccatgg taaagagtgt      60

tataatg                                                              67


<210>  4
<211>  67
<212>  DNA
<213>  Artificial

<220>
<223>  r0-bottom

<400>  4
gatccattat aacactcttt accatggttc gaggaactcg aatccatggg taacctccgt      60

tataatg                                                              67


<210>  5
<211>  67
<212>  DNA
<213>  Artificial

<220>
<223>  r1-top

<400>  5
aattccgtct aactcatcgc gcgtgaataa acctcttgga ggttattcac cgcaaaatgt      60

tagacgg                                                              67


<210>  6
<211>  67
<212>  DNA
<213>  Artificial

<220>
<223>  r1-bottom

<400>  6
gatcccgtct aacattttgc ggtgaataac ctccaagagg tttattcacg cgcgatgagt      60

tagacgg                                                              67


<210>  7
<211>  67
<212>  DNA
<213>  Artificial

<220>
<223>  r2-top

<400>  7
aattcagggt aacgctacgc gcagtgccaa gcatctatga tgctggcact cgcctcttgt      60

taccctg                                                              67


<210>  8
<211>  67
<212>  DNA
<213>  Artificial

<220>
<223>  r2-bottom

<400>  8
gatccagggt aacaagaggc gagtgccagc atcatagatg cttggcactg cgcgtagcgt      60

taccctg                                                                67


<210>  9
<211>  67
<212>  DNA
<213>  Artificial

<220>
<223>  r3-top

<400>  9
aattctctct aacctgctac tctatagtac agtaaggttt actgactata agtcgggtgt      60

tagagag                                                                67


<210>  10
<211>  67
<212>  DNA
<213>  Artificial

<220>
<223>  r3-bottom

<400>  10
gatcctctct aacacccgac ttatagtcag taaaccttac tgtactatag agtagcaggt      60

tagagag                                                                67


<210>  11
<211>  67
<212>  DNA
<213>  Artificial

<220>
<223>  r4-top

<400>  11
aattcagcgt aacaattgtg actgattaag caagcccagc ttgctaatca tcaagcacgt      60

tacgctg                                                                67


<210>  12
<211>  67
<212>  DNA
<213>  Artificial

<220>
<223>  r4-bottom

<400>  12
gatccagcgt aacgtgcttg atgattagca agctgggctt gcttaatcag tcacaattgt      60

tacgctg                                                                67

<210>  13
<211>  67
<212>  DNA
<213>  Artificial

<220>
<223>  r5-top

<400>  13
aattctttct aactccgcca acccggagaa gcgtggccca cgctctccgg ttgatattgt      60

tagaaag                                                                67


<210>  14
<211>  67
<212>  DNA
<213>  Artificial

<220>
<223>  r5-bottom

<400>  14
gatcctttct aacaatatca accggagagc gtgggccacg cttctccggg ttggcggagt      60

tagaaag                                                                67


<210>  15
<211>  67
<212>  DNA
<213>  Artificial

<220>
<223>  r6-top

<400>  15
aattcgcctt aacaatacag gctatgttat ttggtcggac caaaaacata cctagtaggt      60

taaggcg                                                                67


<210>  16
<211>  67
<212>  DNA
<213>  Artificial

<220>
<223>  r6-bottom

<400>  16
gatccgcctt aacctactag gtatgttttt ggtccgacca aataacatag cctgtattgt      60

taaggcg                                                                67


<210>  17
<211>  67
<212>  DNA
<213>  Artificial

<220>
<223>  r7-top

<400>  17

aattcatctt aactgcttac acccgggcaa cctttcctaa aggtgcccgg tgtggcttgt     60

taagatg                                                               67


<210>  18
<211>  67
<212>  DNA
<213>  Artificial

<220>
<223>  r7-bottom

<400>  18
gatccatctt aacaagccac accgggcacc tttaggaaag gttgcccggg tgtaagcagt     60

taagatg                                                               67


<210>  19
<211>  82
<212>  DNA
<213>  Artificial

<220>
<223>  rdmAttC0


<220>
<221>  misc_feature
<222>  (23)..(26)
<223>  n is a, c, g, or t

<220>
<221>  misc_feature
<222>  (56)..(59)
<223>  n is a, c, g, or t

<400>  19
gatcggatcc attataacac tcnnnnccat ggttcgacga actdgaatcc atgggnnnnc     60

tccgttataa tgaattcacc ag                                              82


<210>  20
<211>  23
<212>  DNA
<213>  Artificial

<220>
<223>  primerrdmAttC0

<400>  20
ctggtgaatt cattataacg gag                                             23


<210>  21
<211>  24
<212>  DNA
<213>  Artificial

<220>
<223>  MRV

<400>  21

agcggataac aatttcacac agga                                          24


<210>  22
<211>  19
<212>  DNA
<213>  Artificial

<220>
<223>  MRVD2

<400>  22
ttctgctgac gcaccggtg                                                 19


<210>  23
<211>  23
<212>  DNA
<213>  Artificial

<220>
<223>  SWbegin

<400>  23
ccgtcacagg tatttattcg gcg                                            23


<210>  24
<211>  24
<212>  DNA
<213>  Artificial

<220>
<223>  SWend

<400>  24
cctcactaaa gggaacaaaa gctg                                           24


<210>  25
<211>  29
<212>  DNA
<213>  Artificial

<220>
<223>  BamHI-pSUlib-F

<400>  25
tttggatcca ctagtagcgg ccgctgcag                                      29


<210>  26
<211>  33
<212>  DNA
<213>  Artificial

<220>
<223>  attI0

<400>  26
tttggatcca ttataacttt gttttagggc gac                                 33

## Description

The present invention relates to the fields of molecular biology and biotechnology and to *de novo* integron recombination sites and the use of these. In particular the present invention relates to isolated polynucleotides encoding synthetic attC sites as well as vectors comprising such polynucleotides, the use of such materials and methods to generate and test synthetic attC sites.

Site specific DNA recombination is one of the basic tools of genetic engineering. It allows for the efficient combination of heterologous DNA sequences into expression vectors, the integration of synthetic constructs into the genome of host organisms, the manipulation of large DNA fragments directly *in vivo* and much more.

All the site specific DNA recombination tools developed to date are derived from natural recombination systems, which have a well-defined recombination site whose sequence cannot be changed. This severely limits the flexibility and number of applications of such site specific recombination systems.

The basis of these different systems is site-specific recombinases which are proteins that are present in many organisms such as viruses and bacteria and which are characterized as having both endonuclease and ligase properties. These recombinases (along with associated proteins in some cases) recognize specific DNA sequences and exchange the nucleic acid segments flanking these sequences with those from a donor nucleic acid.

A type of genetic element that can be used as a site specific DNA recombination tool are integrons which have the further useful properties of being able to capture and rearrange gene cassettes and to ensure their correct expression. These were first identified in the 1980s from clinical isolates of Gram negative bacteria that showed antibiotic multiresistance. Integrons were thought to be mobile genetic elements able to gather genes encoding antibiotic resistance [1, 2]. Nowadays it is confirmed that these "mobile integrons" (MIs) found on transposons and plasmids are the major vectors for antibiotic resistance in Gram-negative bacteria [3] and occasionally also in Gram positive bacteria [4]. The MIs were divided into five groups according to their encoded integrase. However, in the late 1990s another class of sedentary integrons was discovered in bacterial genomes [5, 6] that mostly contain host-specific genes of unknown functions, as well as genes encoding proteins involved in adaptive functions [7-10]. There is evidence that these "chromosomal integrons" (CIs) are the evolutionary source of the MIs [11, 12]. Integrons are now considered as a general gene-capture system used in bacterial adaptation [7, 13].

Common to all integrons are a stable, nonmobile portion encoding the functional elements located upstream of a variable array of mostly promoterless gene cassettes. This stable platform contains the gene for the integron integrase IntI and its promoter Pᵢₙₜ. Directly adjacent to the integrase but orientated in the opposite direction is the primary recombination site for the gene cassettes attI [14]. The strong promoter for cassette expression P_{c} is encoded in the open reading frame of IntI but in the opposite direction, oriented towards the integration point attI [15-17]. A gene cassette is a circular exogenous non-replicative DNA element, composed of an open reading frame (ORF) followed by the cassette recombination site attC that is recognized by IntI [18-20]. IntI catalyzes the integration of attC containing cassettes at attI leading to their expression by the promoter P_{c} following integration. Consecutive insertions can lead to the formation of a cassette array comprising multiple ORFs and this constitutes the variable part of the integron [14, 21]. IntI catalyzed recombination of two attC-sites leads to the excision and recircularization of cassettes [22]

The integron integrases belong to the family of tyrosine recombinases [23], with members like lambda, Cre, Flp, XerC/XerD or FimB/FimE [24]. Members of this family are able to integrate, inverse or excise defined DNA segments by site specific recombination. In this process the DNA is broken and rejoined without DNA synthesis and high energy cofactors [25]. The different steps of the recombination process are: the recombinase binds to the two recombination sites as dimers, so that a synaptic complex is built by two strands of DNA and four recombinase monomers. Two of the four monomers, located diagonally in the complex, initiate the process by cleavage of the bound DNA strand. The phosphodiester energy is thereby conserved by the establishment of a DNA-protein-covalent bond of the 3'-phosphate with a nucleophilic tyrosine side chain, exposing the 5'-hydroxy group. In a next step, the phosphotyrosine is attacked by the free hydroxy group of the other strand, leading to an intermediate structure called a Holliday Junction. In the subsequent isomerization process, the two initially inactive recombinases become active and vice versa. The process of DNA breakage and rejoining with the neighboring strand is repeated, which solves the junction and results in a strand exchange.

The typical core recombination site of tyrosine recombinases is composed of highly conserved 9-13 base pairs (bp) inverted binding sites separated by a 6-8 bp central region [25]. AttI sites differ from this organization and although there is no sequence consensus of attI sites in different integron classes [12], their structure is similar: two sites referred to as L and R boxes constitute the integrase binding sites, where the L box is always degenerate with respect to the R box. The central region differs greatly between different attI sites. The recombination point can be found in a conserved 5'-GTT-3' triplet in the R box, where the strand transfer occurs between G and T [14, 42].

The structure of the attC sites are more complex although they all share a palindromic architecture. This property allows attC sites to establish a cruciform formation by extrusion and self pairing of the double strands or hairpin formation in case of ssDNA [14, 20, 46].

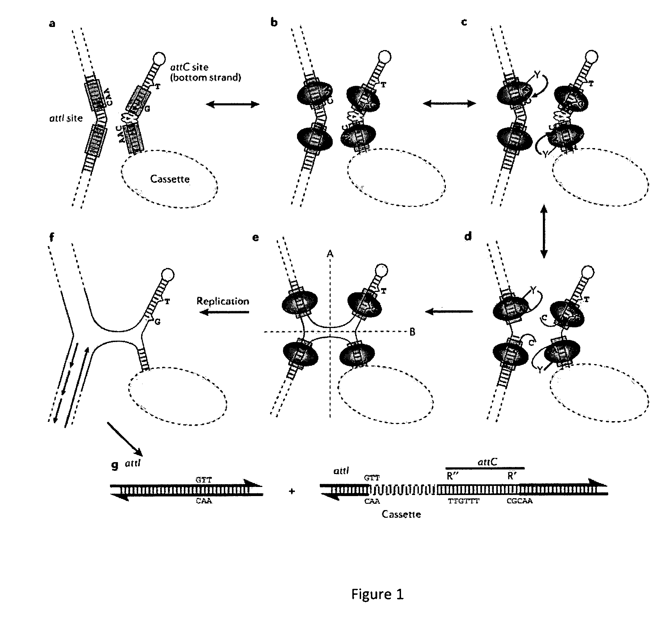

The integron integrase recombines the double stranded (ds) attI with a ss attC that is folded to a dsDNA-like cruciform formation. A possible pathway for the recombination of a single stranded and a double stranded substrate is shown in Figure 1. The initial steps are identical to the normal recombination procedure of tyrosine recombinases discussed above. Two integrase monomers bind to the core sites of both the attI and the attC (Figure 1b). Two opposing monomers become active and nick one strand, which results in the formation of a Holliday Junction (Figure 1c, d). From this point on, the proposed recombination mechanism of the IntI differs from that of other recombinase family members. The classical resolution of the complex through the A axis would result in the original sites, while a cut at the B axis would lead to covalently closed linear DNA products, which are abortive. To prevent this occurring it has been proposed a replication step takes place (Figure 1 f), that bypasses the strand exchange by the isomerization process. This model requires the termination of integrase activity after the first strand exchange to prevent the second one.

At the present time however the use of integrons as the basis of a recombination system is extremely limited due to the inherent limitations of being able to only use predefined sequences as the attI and attC components of such a system.

The inventors have now developed recombination sites based on the bacterial integron recombination system that have a much looser sequence requirement than any system used so far. The inventors have shown unexpectedly that integron *attC* recombination sites have very few required sequence elements and are recognized by the integron integrase based on their secondary structure features as a folded single strand of DNA. This remarkable property has allowed the inventors to rewrite the attC site primary sequence to encrypt in them a second function such as transcription promoters, protein binding sites, in-frame protein linkers, or merely to alter the sequence so as to make its cloning and manipulation easier; these types of de novo attC sites therefore have infinite applications in biotechnology.

Therefore in accordance with a first aspect of the present invention there is provided an isolated DNA single strand consisting of a sequence comprising at least the elements:
N₁-N₂-N₃-N₄-N₅-N₆-N₇-N₈-N₉ (SEQ ID NO: 1) in the order specified;
wherein N₁ comprises at least 4 nucleotides wherein the third from last nucleotide is A and N₁ is complementary to N₉, N₂ if present comprises no more than 2 nucleotides, N₃ comprises 6 to 10 nucleotides and is non-complementary to N₇; N₄ comprises 9 to 30 nucleotides and is complementary to N₆; N₅ comprises 3 to 50 nucleotides; N₆ comprises 9 to 30 nucleotides and is complementary to N₄ which comprises at least one nucleotide which forms an extra helical base pair when N₄ and N₆ form a double helical strand; N₇ comprises 6 to 10 nucleotides and is non-complementary to N₃; N₈ if present comprises no more than 2 nucleotides; N₉ comprises at least 4 nucleotides wherein the second from last nucleotide is T and N₉ is complementary to N₁; and wherein said isolated DNA molecule forms a single stranded cruciform structure.

In the present invention a nucleotide unless specified, may be any naturally occurring or artificial nucleotides or molecules able to mimic the properties of nucleotides. Examples of naturally occurring nucleotides include adenosine, thymine, guanine and cytosine. Examples of artificial nucleotides include isoguanine, isocytine, 2-amino-6-(2-thienyl) purine, pyrrole-2-carbaldehyde, 2'-deoxyinosine (hypoxanthine deoxynucleotide) derivatives. Examples of molecules that can mimic the properties of nucleotides include metal coordinated bases, such as two 2,6-bis(ethylthiomethyl)pyridine (SPy) with a silver ion or pyridine-2,6-dicarboxamide (Dipam) and a mondentate pyridine (Py) with a copper ions, nitroazole analogues and hydrophobic aromatic non-hydrogen-bonding bases.

As indicated above the inventors have now found that the sequence of an attC site can be massively altered so long as certain key features are retained and the final structure of the lowest energy single strand form of the DNA molecule have the following features.

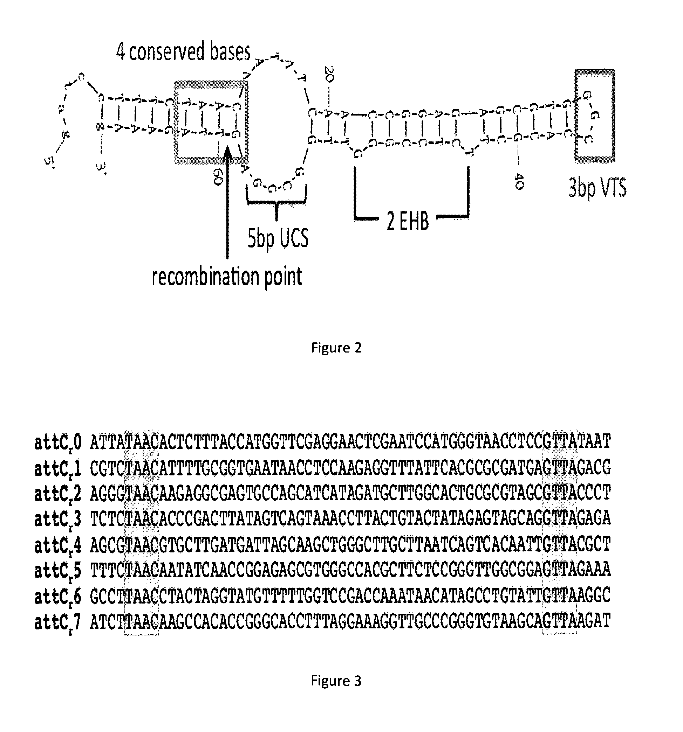

With reference to Figure 2 these conserved features in the single stranded cruciform structure are (i) at least a complementary four base pair sequence nAnn - nnTn positioned at either end of the strand and which form a double stranded structure; (ii) a further complementary stretch of sequence which if present comprises up to 2 nucleotides; (iii) an unpaired central spacer (UCS); (iv) a stretch of complementary sequences with (v) at least one 3 extrahelical base pair (EHB) on the lower (or most 3' strand); (vi) a variable terminal structure (VTS).

These conserved features correspond as follows to the features of SEQ ID NO: 1:
(i) & (ii) Corresponds to the beginning and end portions of SEQ ID NO: 1 N₁/N₉ and N₂/N₈ (if present) which form a continuous double stranded structure;
(iii) Corresponds to N₃ & N₇;
(iv) Corresponds to N₄ & N₆ which form a double stranded structure;
(v) Corresponds to the at least one nucleotide in N₆ that forms an EHB when N₄ & N₆ form a double helix
(vi) Corresponds to N₅.

In the present invention when a sequence is described as being complementary this means that it is able to form a double stranded helical structure with a sequence to which it is described as being complementary.

In the present invention when a sequence is described as being non-complementary this means that it is unable to form a double stranded structure with a sequence to which it is described as being non-complementary with. This does not mean however that small complementary regions cannot be present with the overall molecule, but as these are interspersed with non-complementary regions no stable double stranded structure is able to form.

When elements are described as being before/after other elements or the first/last on the sequence this refers to the order upon the claimed single stranded DNA molecule moving in a 5' to 3' direction.

Based upon these principles, the inventors have found it to be possible to generate an attC site which comprises all of the required functional and structural elements so that a sequence associated with it can be introduced by recombination to an attI site, but which may also comprise other genetic elements and operators so as to provide additional functionality.

In accordance with a further aspect of the present invention portions N₃ & N₇ comprise the same number of nucleotides.

Alternatively portions N₃ & N₇ comprise a different number of nucleotides.

In accordance with a further aspect of the present invention portion N₆ may comprise at least one additional EHB.

In accordance with a further aspect of the present invention each said EHB is spaced at least 3 nucleotides apart from any other present.

In accordance with a further aspect of the present invention there are 8 to 14 nucleotides between the last EHB and the first nucleotide of portion N₉.

In accordance with a further aspect of the present invention these *de novo* attC sites may encode transcription promoters or protein binding sites or in-frame protein linkers.

As the attC sites can now encode a diverse range of additional functions, these properties in combination with the natural ability of integrons to shuffle, delete and rearrange gene cassettes, means that these synthetic attC sites could become a common tool in synthetic biology. By designing open reading frames of gene cassettes where the recombination site is part of the code, one can use the synthetic integron in a wide variety of techniques such as a Plug and Play method.

In this context "Plug and Play" means a combinatorial approach based on a process of decoupling, that is breaking a complex problem such as the generation of a new enzyme with altered kinetics and substrate affinity into its constituent parts so as to make finding the solution easier. To achieve this, the system being studied is divided into different subcomponents that are then shuffled and reassembled in different orders. An example of the Plug and Play method is gene shuffling in protein engineering leading to higher stability and better performance of the resultant proteins.

Based upon the natural propensity of integrons to randomly shuffle and rearrange the ORFs which are recombined into the attI site, if these ORFs are associated with a *de novo* attC site that encoded a protein linker, the process of shuffling the protein domains would occur automatically as the various members of a library of these ORFs were inserted into a plasmid containing the attI site.

To give an example of such a method, it would be of great interest to use the synthetic integron in a combinatorial approach to engineer non-ribosomal polyketide and polypeptide synthetases. These complex proteins are composed of series of functional modules that each catalyze the addition of a specific monomer on a growing polyketide/polypeptide chain. The thereby produced molecules are often of pharmaceutical interest [104]. As the order of the protein domains of the synthetases determines the output molecule [105], the synthetic integron could be used to shuffle these domains leading to the production of novel, biologically active compounds.

Another advantage of such a system is that a frame shift mutation is very unlikely to occur, as the gene cassettes have clearly defined borders and the recombination occurs only at a specific site.

In accordance with another aspect of the present invention there is provided a vector comprising the DNA molecule sequence consisting of:
TAACN₃N₄N₅N₆N₇GTTA (SEQ ID NO: 2).

Wherein each of portions N₃, N₄, N₅, N₆ & N₇ are as provided in the definitions above.

In accordance with a further aspect of the present invention the vector also comprises a multiple cloning site (MCS) into which an ORF of interest may be cloned so that following insertion by site specific recombination of the ORF into an attI site the ORF of interest is expressed.

According to a further aspect of the present invention there is provided the use of a DNA molecule sequence consisting of SEQ ID NO: 1 or SEQ ID NO: 2 or a vector comprising either of said DNA molecule to introduce by site specific recombination a DNA sequence of interest into a preselected DNA site.

According to a further aspect of the present invention there is provided a method to generate a *de novo* attC site comprising the steps:
a) Generating a library of size 2N of putative attC sites each of which comprises a sequence N₁-N₂-N₃-N₄-N₅-N₆-N₇-N₈-N₉ (SEQ ID NO: 1);
b) Calculate a score for each attC site using a scoring function specific to the intended secondary function of the attC site;
c) Ranking the attC sites according to their score;
d) Discarding the bottom N attC sites;
e) Duplicating each of the top N attC sites and in each of the duplicates randomly mutating at least one nucleotide therein, verifying that the resulting attC site still comprises a sequence N₁-N₂-N₃-N₄-N₅-N₆-N₇-N₈-N₉ (SEQ ID NO: 1);
f) Repeating steps b) to e) until an end condition is met.

The method detailed can be adapted so as to generate attC sites that have a secondary function such as a promoter, protein attachment site, protein linker, recombination site of another recombination system (e.g. Frt/Flp, XerCD, Lambda Integrase etc.), transcription terminator, ribosome binding site, phage encapsidation sequence, replication origin, origin of transfer, this is an non-exhaustive list.

For instance when it is intended that the *de novo* attC site encodes a disordered neutral protein linker an adapted method as follows could be used:
a) A population of size 2N random attC sites is generated each of which comprises SEQ ID NO: 1.
b) A score is computed for each attC site of the population using a scoring function as follows:
   the attC is translated (if the length of the attC is not a multiple of 3 then the position of the attC site is used to determine the first amino acid of the peptide);
   if there is a stop codon in the peptide the returned score is Score=-100, Else the a score is computed as follow;
      the IUPred function (106 &107) is used to determine the disorder tendency of each amino acid of the peptide
      a peptide disorder score Sd is computed as the sum of the disorder tendency of all amino acids (highly disordered peptides will have a higher score)
      the charge of each amino acid of the peptide is computed with the 'charge' function of the EMBOSS package
      a neutrality score Sn is computed as the opposite of the sum of the absolute values of all amino acids (it is a negative number that penalizes charged peptides)
      a proline score Sp is computed as the opposite of the number of prolines in the peptide (it is a negative number that penalizes peptides with prolines)
      the final score returned by the function is a weighted average of the previous scores:
   Sd/20+Sn/2+Sp/10.
c) The attC sites are ranked according to their scores and the N best sites are duplicated;
d) The duplicated sites are randomly mutated as follow:
   - a position of the attC site is randomly chosen so that is not the conserved A nucleotide N₁, or the conserved Y nucleotide in N₉ or any of the EHBs in N₆
   - the base is randomly changed to any of the 3 other possible bases
   - if the base that is chosen is supposed to be paired with a base in another segment, then the corresponding base is changed as well to keep the complementarity
e) We go back to step 2 until the score of the best site does not improve in more than 100 rounds.

Alternatively a method to generate a attC site which also encodes a selected nucleotide sequence such as a promoter or replication origin, which comprises the steps:
a) Generating a population of putative attC sites each of which comprises a sequence consisting of SEQ ID NO: 1;
b) Inputting a selected sequence which the finalized attC site should be as identical to as possible. An example of an inputted sequence would be a promoter sequence.
c) Specifying in the selected sequence those bases that need to be conserved ("unmutables") so that the sequence continues to have its function. These unmutable residues would normally have been shown to be essential to the function of the respective element in previous studies concerning the starter sequence and their mutation will therefore be penalized by the method. As the exact relation between sequence and function is often not completely elucidated, the method is performed in a way that it also keeps as many residues as possible of the "mutable" bases of the input sequence. This property increases the chance to obtain a functional site in the end.
d) Assigning a score to each of the attCr sites in the population using the following scoring scheme. Iterating over each base of each attCr site to checks if its position is the position of an unmutable in the input sequence. If so, the variable "u" that counts the number of unmutables is raised by one. Subsequently, the program considers the actual bases at that position in the two sequences. If both are the same base, the score for the unmutable bases "sᵤ" is raised by one, if they differ, the score remains unchanged. The same procedure is done in case the considered base of the attCr site is not an unmutable. The only difference here is that the variable "v" is raised by one, counting the number of "mutable bases", and the according score is "Sᵥ".
e) A final score for each putative attCr in the library is calculated. Therefore, the number of conserved residues is normalized by the total number of residues for both, the unmutables (sᵤ/u) and mutable residues (sᵥ/v ). Since the conservation of unmutables is more important than that of other residues, the sᵤ/u is multiplied by ten and the product is added to sᵥ/v resulting in the final score of the attC site. This scoring system rewards both, the conservation of unmutables as well as of all other residues of the input sequence of choice; however, the reward for a conserved unmutable is ten times higher.
f) The population of attCr sites is then sorted by their score and only the top 50% of the sequences are kept.
g) These kept sequences are duplicated and each duplicate undergoes a random mutation procedure. Subsequently, the created population is scored again against the selected sequence.
h) The cycle of evaluation, selection and mutation (steps a) to g)) is repeated T times (T can be chosen by the user) and in the end, the method returns the highest scoring sequences.

Normally this method is performed using a computer.

According to a further aspect of the present invention there is provided a method to test the properties of a *de novo* attC site according to the present invention, comprises the steps of:
a) Introducing into a conjugation competent donor strain of bacteria a first plasmid comprising the attC site to be tested in combination with a first resistance marker, wherein said first plasmid is suitable for conjugational transfer and wherein said donor strain is an auxotroph;
b) Preparing a recipient strain comprising a second plasmid containing the ORF of the intl integrase and a second resistance marker and a third plasmid containing the attI recombination which also comprises a third resistance marker;
c) The donor and recipient strains are then mixed under conditions wherein conjugation occurs between them;
d) Growing the resulting mixed population under selection conditions designed to eliminate the auxotrophic donor strain and to test for either the presence of the first selection marker or the second selection marker;
e) Comparing the ratio of the populations showing resistance to the first selection marker versus those showing resistance to the second selection marker so as to calculate the recombination frequency of the tested attC site.

In accordance with a further aspect of the present invention the methods of designing and testing *de novo* attC sites can be combined, such that attC sites generated according to the method above are then tested using the specified method and those which show the highest frequency of recombination are then isolated and re-inputted as the starting pool of attC sites from which the next generation of attC sites are derived. This cycling between the methods can continue until such time as the frequency of recombination does not improve any further.

Another application is to use the experimental results to improve the model of the attC site and generate sequences that will recombine more reliably. This would involve changing the definition of attC sites and possibly adding a second scoring function to the algorithm that would estimate the ability of the site to recombine. The final score of a given sequence could be a weighted average of the two scoring functions.

For a better understanding of the invention and to show how the same may be carried into effect, there will now be shown by way of example only, specific embodiments, methods and processes according to the present invention with reference to the accompanying drawings in which:
Figure 1 shows a model of integron mediated recombination a) Cassette integration in integrons occurs by recombination of a dsattI and a ssattC site, that folds into a DNA-like structure by intrastrand basepairing. b) The initial steps are identical to the normal recombination between two dsDNA molecules: Four integrase monomers assemble at the recombination site, two monomers are active and cut one strand of DNA (c) and the subsequent strand transfer (d) leads to the establishment of a Holliday junction (e). Since resolution along the A-axis would lead to the initial molecules, while resolution along the B-axis would result in a covalently closed abortive DNA double strand, a second strand transfer is not undertaken. Instead, the junction is solved by a replication step (f) that synthesizes two products seen in (g), one being the integrated gene cassette.
Figure 2 shows the conserved features of the random attCr sites. The four conserved bases of the recombination site are maintained, as well as a 5 bp unpaired central spacer (UCS) and the extrahelical bases (EHB) G and T at specific positions. The variable terminal structure (VTS) was kept small, comprising only three nucleotides.
Figure 3 shows multiple sequence alignment of the bs random attCr sites. The alignment underlines the great variations in primary sequence of the designed sites. Only six nucleotides are maintained in all sequences: The recombination point TAAC/GTTA is shown in cyan and the two extrahelical bases in yellow.

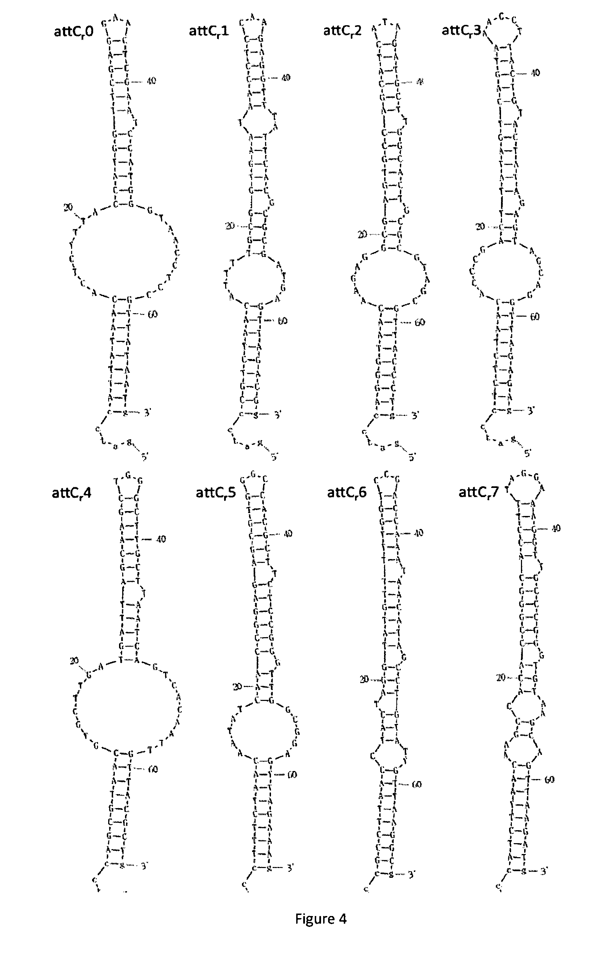

Figure 4 shows the structure of the bottom strand of random attCr sites.

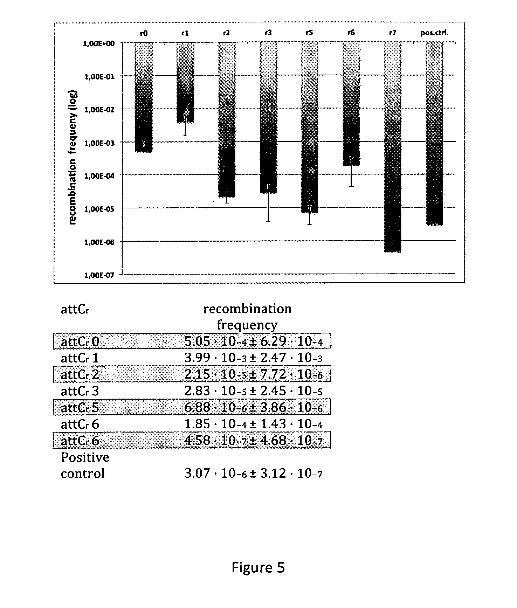

Figure 5 shows the recombination frequencies of random attCr sites. The positive control used is the attC V CR2=1, N is 3 and the error depicted is the standard error of the mean (SEM).

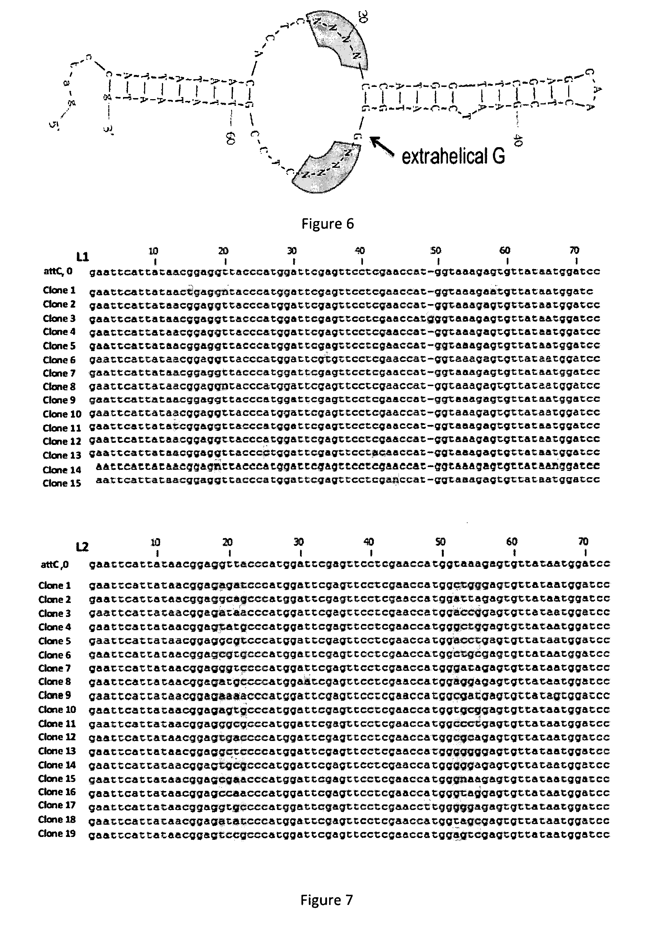

Figure 6 shows Design of library 2. In order to elucidate if the deformed UCS and the lacking EHB is limiting the recombination efficiency, only the eight bases shown in red are mutated.
Figure 7 Alignment of L1 and L2 with original attCr 0. The alignment of L1 (upper alignment) reveals that only around 30% of the sequences carry a mutation, whereas in library 2 (lower alignment) all sequences are different.

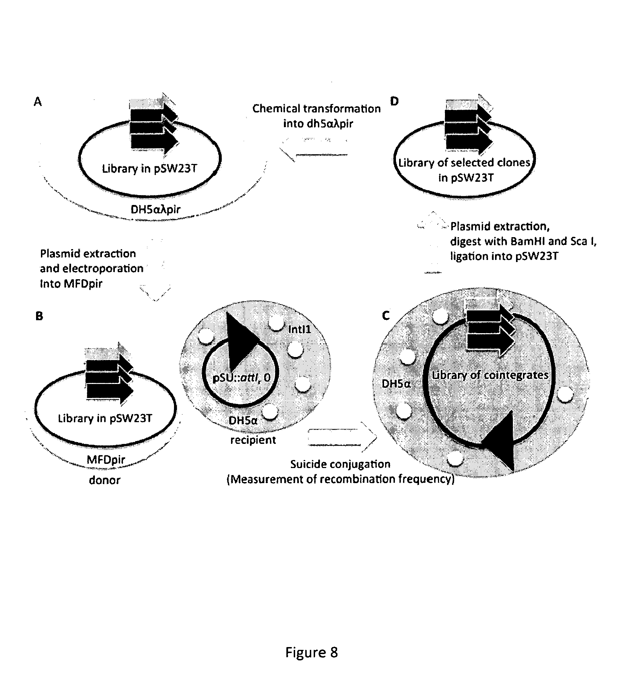

Figure 8 Selection for mutants with high recombination frequency A) The libraries of mutants of attCr 0 are first transformed into a pir+ cloning strain DH5α pir. B) The suicide conjugation assay however is done with strain containing the conjugation machinery (MFDpir). This strain recombines with the recipient DH5α strain expressing the integrase (yellow dots) and carrying the attIr 0 recombination site on a pSU plasmid. C) After recombination, the cointegrates are extracted from the recipient strain. D) The attC sites are recovered and ligated into their original vector. The reestablished libraries, enriched of mutants with high recombination frequencies, are then transformed into DH5α pir and (A) the cycle is repeated until the recombination frequency does not further increase.

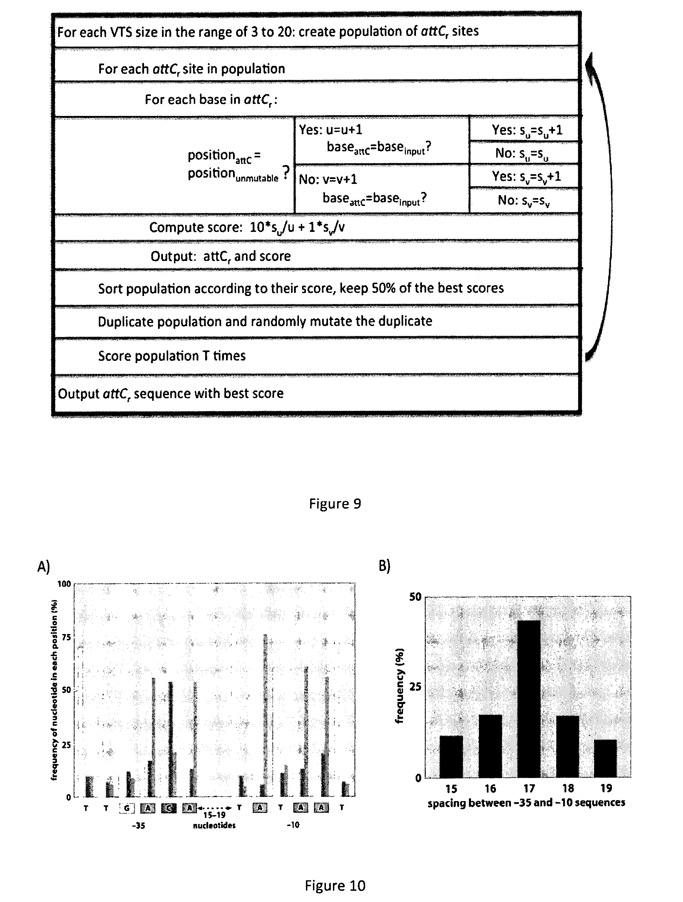

Figure 9 shows a flowchart of algorithm that computes coding attC sites The program needs the sequence of choice as an input as well as the positions of the bases that need to be conserved in order to maintain functionality. The program refers to these bases as "unmutables".
Figure 10 shows the consensus sequence of E. coli promoters. A) The nucleotide composition of the -10 and -35 regions are obtained by the comparison of 300 promoters. The consensus sequence shown below the graph represents the most common nucleotide found at a certain position in the collection of promoters. B) The distribution of spacing between the two regions.

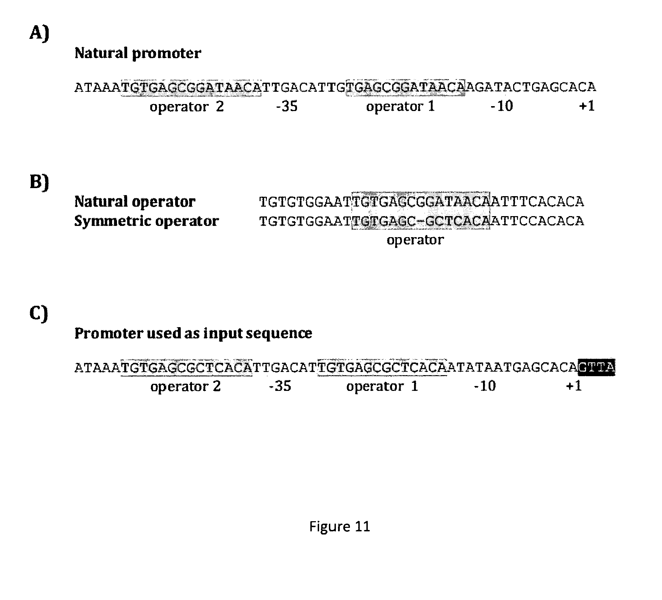

Figure 11 shows the derivation of the input sequence for the lac promoter-attC site A) The wildtype lac promoter is shown with the -10 and -35 region as well as the transcriptional start site (+1) depicted in yellow and the operators 1 and 2 boxed in blue. B) Comparison of the wild-type operator and the perfectly symmetric operator with a 10-fold higher binding affinity of the lac repressor. C) Input sequence used for the computing of a lac promoter-attC site: the operators are symmetric and the -10 and -35 regions are changed to the most conserved ones for E. coli promoters. The operator 2 is not defined as an unmutable to prevent unwanted secondary structure formation. The recombination site GTTA underlined in red is located immediately downstream of the transcription start site so that that the cassette downstream of the promoter-attC site is being transcribed upon recombination and derepression.

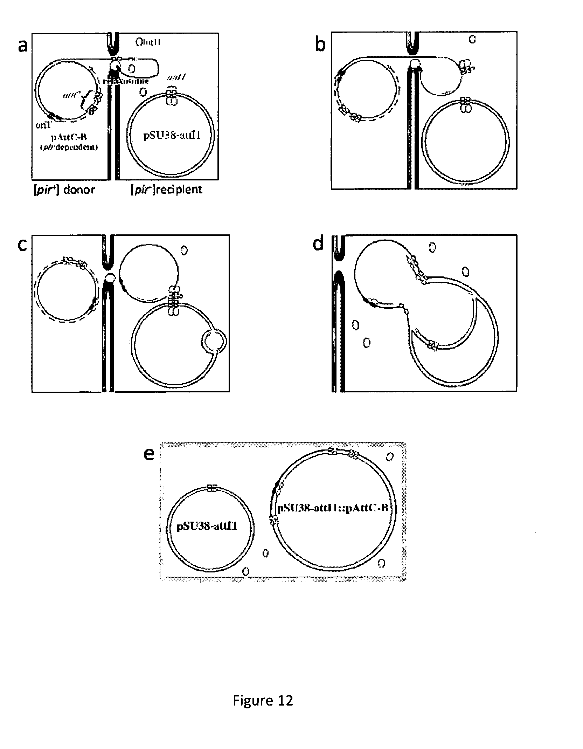

Figure 12 shows Suicide conjugation assay a) The pSW plasmid carrying the attC site is delivered as a single strand by conjugation from the pir+ donor to the pir⁻ recipient cell, where it cannot replicate. b) The recipient cell expresses the integrase that recognizes and binds both the attC site of the delivered plasmid and the attI site of its pSU plasmid. c) The catalyzed attI x attC recombination integrates the pSW plasmid into the pSU plasmid leading to the formation of a cointegrate (d). e) After conjugation, the recipent cell contains two pSU-derived plasmids, the original one and the cointegrate.

### Materials and Methods

### Suicide conjugation assay

The starting point of the assay are two bacterial cultures, donor and recipient strains. The donor strain contains the attC recombination site on an R6K-derived plasmid of the pSW family. The plasmid carries an oriVR6K origin of replication, that is dependent on the π-protein for replication and an oriTRP4 origin of transfer, that renders the plasmid suitable for conjugational transfer. The orientation of oriTRP4 determines the strand that will be transferred. The plasmid also provides chloramphenicol (Cm) resistance. The genome of the donor strain MFDpir carries the pir gene that codes for the π-protein relevant for plasmid replication, as well as the transfer machinery for the conjugation process. Moreover, MFDpir is auxotroph for diaminopimelic acid (DAP) to crosslink peptidoglycans in its cell wall [49,100].

The recipient strain contains two important plasmids: one pSU-derived plasmid expressing the integrase and providing ampicillin (Amp) resistance and one plasmid containing the attI recombination target where recombination with the transferred attC site occurs. The latter plasmid has a kanamycin resistance gene. The E.coli strain containing these two plasmids in the suicide conjugation assay is DH5α.

Figure 12 depicts the recombination process upon contact of donor and recipient strain. The donor strain transfers a single strand of the pSW plasmid into the recipient cell. Since the recipient strain does not provide the π protein, the plasmid cannot be replicated. However, the attC site of the pSW plasmid can recombine with the attI site of the pSU plasmid as illustrated in Figure 12.

Therefore, the pSW plasmid is integrated into the pSU plasmid resulting in a cointegrate.

The recombination frequency of the different attC sites is the ratio of the number of colonies that grow on Cm after conjugation and the number of colonies that grow on Amp. The rationale behind this calculation is that neither the donor strain, as it depends on DAP, nor the recipient strain, as it does not have chloramphenicol resistance, can grow on Cm plates. The only cells that are able to do so are those where attI x attC recombination happened. These cells have the cointegrate that carries the Cm resistance gene of the pSW plasmid. Dividing by the number of Amp resistant cells normalizes the number of recombination events by the number of available recipient cells.

### Construction of random attCr sites

Seven random attCr sites were constructed by annealing complementary, partially overlapping primers (top and bottom), listed in Table 1, after phosphorylation of their 5'-ends by polynucleotide kinase. The annealing was achieved by incubation at 90 °C for five minutes and subsequent cooling at room temperature.

The non-overlapping ends of the primers constitute EcoRI and BamHI restriction sites, so that the hybridized oligos can be directly cloned into p135 (see Table 5) linearized by EcoRI and BamHI. The sequences of the resulting plasmids (p9276 - p9282) of the attCr sites was confirmed by sequencing using SWbeg as a primer (Table 4).

### Suicide conjugation assay with random attCr sites

The attCr plasmids (p9276 - p9282) were transformed into MFDpir cells (see Table 3), as was the positive control site attC PCR2/1 (plasmid p9468).

These cells were used as the donor cells in the establishment of recombination frequencies. The recipient strain in all cases was DH5α cotransformed with p929 carrying the attI and p1394 expressing the integrase upon Isopropyl-β-D-thiogalactopyranoside (IPTG) induction. The cultures were grown overnight in the presence of Cm and DAP for the donor strains and Amp, Kan and IPTG for the recipient strain. The overnight cultures were then diluted 1:100 in LB media containing only DAP for the donor strains or IPTG for the recipient cells.

The cultures were grown to an optical density of 0.4 to 0.7 at 600nm wavelength (OD₆₀₀). 1 ml of the respective donor strain was then mixed with one ml of the recipient strain and incubated for four hours at 37 °C on a nitrocellulose filter (Millipore) on a LB agar plate containing DAP. After the incubation, the cells were resuspended in 4 ml of LB media. A serial dilution of the resulting cell suspension was prepared with LB media ranging from 10⁻¹ to 10⁻⁵. 100 µl of the suspensions were plated on agar plates containing either Cm or Amp to select for cointegrates and recipient cells, respectively. The cointegrates were checked for correct recombination by PCR with SWend and MRV as well as by sequencing with SWbeg (see sequences of primers in Table 4).

### Directed Evolution of attCᵣ 0

The directed evolution study comprises the construction of the libraries, the construction of the attIᵣ 0 site as well as the selection for sites with the best recombination properties by repeated rounds of the suicide-conjugation assay.

### Construction of libraries of attCᵣ 0 clones

Two libraries of mutated attCᵣ 0 sites (L1 and L2) were constructed in this study by following two different construction approaches.

L1 was created using random PCR mutagenesis. Therefore, the GeneMorphII Random Mutagenesis Kit from Stratagene was used. Depending on the initial template concentration and the number of PCR cycles, it introduces 1 to 16 mutations per kilobase. In order to obtain at least one mutation per attC site, which has a length of 60 bp, a sequential PCR was performed with the highest mutation rate. For this, the initial template concentration was set to 0.1 ng and the number of cycles to 30. After the first run, the product was used as a template in a PCR reaction of 3 cycles with Taq-polymerase, which was thought to repair unfinished strands and increase the quality of the library. This run was followed by a PCR purification and 0.1 ng of the purified product was used for the second mutagenesis PCR under the same conditions as the first. The primers used in all three rounds of PCR are S Wbeg and SWend listed in Table 4.

The product of 904 bp was gel purified and the mutagenized attC sites were excised by ClaI and BamHI resulting in a 84 bp fragment which was again gel purified using the QIAEX II Gel Extraction Kit from Qiagen, which is suitable for the extraction of DNA fragments smaller than 100 bp. The library was subsequently cloned into p135 (see Table 5) and transformed into the cloning strain DH5α pir (see Table 3). The success of the mutagenesis was confirmed by sequencing of 20 randomly picked colonies using SWbeg as a primer.

Finally, the library was transformed into MFDpir cells that were used as the donor strain in the suicide conjugation assay.

L2 was created by purchasing (from Eurofins MWG operon) an oligonucleotide (rdmAttC0 in Table 2) that contains the sequence of the attCr 0 but has eight positions of random bases (N). Furthermore the sequence has restrictions sites for EcoRI and BamHI at the ends. The complementary strands were synthesized by Taq polymerase using the primer primerrdmattC0 (Table 4) as an elongation start. The double stranded, linear product was gel purified and digested by EcoRI and BamHI and ligated without purification into p135 (see Table 5). After transformation into DH5αλpir, the establishment of a library was confirmed by sequencing of 20 randomly picked colonies with SWbeg as a primer. For the use in the recombination assay, the plasmids were extracted by midiprep and cloned into MFDpir.

### Construction of random attIᵣ 0

The random attIr 0 for the directed evolution study was created by reverse polymerase chain reaction (PCR) using p28 (Table 5) as a template and the primers BamHI-pSUlib-F and attI0 (see Table 5.4). Both primers introduce a BamHI restriction site to the linear product that can be thus circularized by digestion with BamHI and subsequent self-ligation. To eliminate the template DNA after the proliferation, the product was digested by DpnI, which only cuts methylated DNA and therefore digests the template but not the newly synthesized DNA. The attIᵣ 0 site was sequenced with MRVD2 as a primer. The obtained plasmid was cotransformed with the plasmid p3938, carrying the integrase, into DH5α cells. The resulting strain was used as recipient cells in the directed evolution assay.

### Selection process

The selection process for the mutant with the best recombination properties was achieved by iterative rounds of suicide conjugation assay and retrieving of the attC sites from the cointegrates. As donor strains, MFDpir cells containing the control sites were used, attC_{VCR2/1}, attCr 0 and the libraries of attCr 0 mutants, L1 and L2 (plasmids p929, p9276, pSWL1 and pSWL2 in Table 5). As a recipient strain DH5α was cotransformed with the pSU plasmid carrying the attIr 0 site (p9383) and the one coding for the integrase IntI1 under control of the pBAD promotor (p3938). The suicide conjugation assay was performed as described above but using arabinose instead of IPTG to induce the expression of IntI1.

After recombination the cointegrates were extracted from the recipient cells by Qiagen midiprep and the attC sites were excised by BamHI and ScaI followed by gel purification. p135 was linearized by ScaI and BamHI and the backbone was gel purified. The recombination sites were ligated overnight at 16 °C into the p135 backbone. The resulting plasmid was transformed by chemical transformation into the pir+ cloning strain DH5α pir. The library was then extracted by midiprep and transformed by electroporation into MFDpir. The resulting cells were used as donor strain in the next round of recombination.

**Table 1**

| **Name** | **Sequence** |
|---|---|
| r0-top | aattcATTATAACGGAGGTTACCCATGGATTCGAGTTCCTCGAACCATGGT AAAGAGTGTTATAATg (SEQ ID NO: 3) |
| r0-bottom | gatccATTATAACACTCTTTACCATGGTTCGAGGAACTCGAATCCATGGG TAACCTCCGTTATAATg (SEQ ID NO: 4) |
| r1-top | aattcCGTCTAACTCATCGCGCGTGAATAAACCTCTTGGAGGTTATTCACC GCAAAATGTTAGACGg (SEQ ID NO: 5) |
| r1-bottom | gatccCGTCTAACATTTTGCGGTGAATAACCTCCAAGAGGTTTATTCACGC GCGATGAGTTAGACGg (SEQ ID NO: 6) |
| r2-top | aattcAGGGTAACGCTACGCGCAGTGCCAAGCATCTATGATGCTGGCACT CGCCTCTTGTTACCCTg (SEQ ID NO: 7) |
| r2-bottom | gatccAGGGTAACAAGAGGCGAGTGCCAGCATCATAGATGCTTGGCACT GCGCGTAGCGTTACCCTg (SEQ ID NO: 8) |
| r3-top | aattcTCTCTAACCTGCTACTCTATAGTACAGTAAGGTTTACTGACTATAA GTCGGGTGTTAGAGAg (SEQ ID NO: 9) |
| r3-bottom | gatccTCTCTAACACCCGACTTATAGTCAGTAAACCTTACTGTACTATAGA GTAGCAGGTTAGAGAg (SEQ ID NO: 10) |
| r4-top | aattcAGCGTAACAATTGTGACTGATTAAGCAAGCCCAGCTTGCTAATCA TCAAGCACGTTACGCTg (SEQ ID NO: 11) |
| r4-bottom | gatccAGCGTAACGTGCTTGATGATTAGCAAGCTGGGCTTGCTTAATCAG TCACAATTGTTACGCTg (SEQ ID NO: 12) |
| r5-top | aattcTTTCTAACTCCGCCAACCCGGAGAAGCGTGGCCCACGCTCTCCGGT TGATATTGTTAGAAAg (SEQ ID NO: 13) |
| r5-bottom | gatccTTTCTAACAATATCAACCGGAGAGCGTGGCCACGCTTCTCCGGG TTGGCGGAGTTAGAAAg (SEQ ID NO: 14) |
| r6-top | aattcGCCTTAACAATACAGGCTATGTTATTTGGTCGGACCAAAAACATA CCTAGTAGGTTAAGGCg (SEQ ID NO: 15) |
| r6-bottom | gatccGCCTTAACCTACTAGGTATGTTTTGGTCCGACCAAATAACATAGC CTGTATTGTTAAGGCg (SEQ ID NO: 16) |
| r7-top | aattcATCTTAACTGCTTACACCCGGGCAACCTTTCCTAAAGGTGCCCGGT GTGGCTTGTTAAGATg (SEQ ID NO: 17) |
| r7-bottom | gatccATCTTAACAAGCCACACCGGGCACCTTTAGGAAAGGTTGCCCGGG TGTAAGCAGTTAAGATg (SEQ ID NO: 18) |

**Table 2**

| **Name** | **Sequence** |
|---|---|
| rdmAttC 0 | GATCGGATCCATTATAACACTCNNNNCCATGGTTCGACGAACT DGAATCCATGGGNNNNCTCCGTTATAATGAATTCACCAG (SEQ ID NO: 19) |
| primerrd mAttC0 | CTGGTGAATTCATTATAACGGAG (SEQ ID NO: 20) |

**Table 3**

| **Strain** | **Genotype** | **Reference** |
|---|---|---|
| DH5α | supE44 ΔlacU169 (∅́801acZ' ΔM15) ΔargF hsdR17 recAl endA1 gyrA96 thi-1 relA1 | Laboratory collection |
| DH5αλpir | DH5α::λpir | [101] |
| MFDpir | MG1655::RP4-2-Tc::[ΔMu1::aac(3)IV -ΔaphA-Δnic35-ΔMu2::zeo] | [100] |

**Table 4**

| **Name** | **Sequence** |
|---|---|
| MRV | AGCGGATAACAATTTCACACAGGA (SEQ ID NO: 21) |
| MRVD2 | TTCTGCTGACGCACCGGTG (SEQ ID NO: 22) |
| SWbegin | CCGTCACAGGTATTTATTCGGCG (SEQ ID NO: 23) |
| SWend | CCTCACTAAAGGGAACAAAAGCTG (SEQ ID NO: 24) |
| BamHI-pSUlib-F | TTTGGATCCACTAGTAGCGGCCGCTGCAG (SEQ ID NO: 25) |
| attI0 | TTTGGATCCATTATAACTTTGTTTTAGGGCGAC (SEQ ID NO: 26) |

**Table 5**

| | **Construction** | **Important characteristics** | **Use** | **Reference** |
|---|---|---|---|---|
| attC sites | | | | |
| pSW23T | pSW23::*ori*T_{RP4} | [Cm^{R}], *oriV_{R6K}, ori*T_{RP4} | vector | [103] |
| p135 | pSW23T::attP; attC; dapA | [CmR], oriVR6K, oriTRP4, attC | backbone of attCr sites | [D. Bikard] |
| p9276 | pSW23T::attP; attCr 0 | [CmR], oriVR6K, oriTRP4, attCr 0 | random attCr 0 | [This work] |
| p9277 | pSW23T::attP; attCr 1 | [CmR], oriVR6K, oriTRP4, attCr 1 | random attCr 1 | [This work] |
| p9278 | pSW23T::attP; attCr 2 | [CmR], oriVR6K, oriTRP4, attCr 2 | random attCr 2 | [This work] |
| p9279 | pSW23T::attP; attCr 3 | [CmR], oriVR6K, oriTRP4, attCr 3 | random attCr 3 | [This work] |
| p9280 | pSW23T::attP; attCr 5 | [CmR], oriVR6K, oriTRP4, attCr 5 | random attCr 5 | [This work] |
| p9281 | pSW23T::attP; attCr 6 | [CmR], oriVR6K, oriTRP4, attCr 6 | random attCr 6 | [This work] |
| p9282 | pSW23T::attP; attCr 7 | [CmR], oriVR6K, oriTRP4, attCr 7 | random attCr 7 | [This work] |
| p9468 | pSW23T::attCV CR2/1 | [CmR], oriVR6K, oriTRP4, attCV CR2/1 | positive control | [87] |
| pSWL1 | pSW23T::attP; L1 | [CmR], oriVR6K, oriTRP4, library 1 | library one | [This work] |
| pSWL2 | pSW23T::attP; L2 | [CmR], oriVR6K, | library two | [This work] |
| | | oriTRP4, library 2 | | |

| **attI sites** | | | | |
|---|---|---|---|---|
| p929 | pSU38Δ::attI1 | [KmR], orip15A, attI1 | attI site in recipient strain | [87] |
| p28 | pSUlib::attI1 | [KmR], orip15A, attI1 | for attIr 0 construction | [D. Bikard] |
| p9383 | pSUlib::rattI 0, BamHI | [KmR], orip15A, attIr 0 | attIr 0 | [This work] |

| **Integrases** | | | | |
|---|---|---|---|---|
| p3938 | pBAD::intI1 | [AmpR], oricolE1, IntI1, arabinose inducible | integrase in library recombination | [88] |
| p1394 | pTRC99A::intI1 | [AmpR], oricolE1, IntI1, IPTG inducible | integrase used in rattCr recombination | [Laboratory collection] |

### Additional information

Sequencing was performed using an ABI BigDye Terminator v.3.1 sequencing kit and an ABI Prism 3100 Capillary GeneticAnalyzer. Oligonucleotides were obtained from Eurofins MWG Operon. E. coli strains were grown in Luria Bertani (LB) at 37°C. Antibiotics were used at the following concentrations: ampicillin (Amp) 100 µg/ml, chloramphenicol (Cm) 25 µg/ml, kanamycin (Kan) 25 µg/ml. Diaminopimelic acid (DAP) was supplemented when necessary at a concentration of 300 µM. For the induction of pBAD or pTRC promoters, 2 mg/ml arabinose or 200 µg/ml IPTG were added, respectively. Standard techniques were used for DNA manipulation and cloning [102]. FastDigest restriction enzymes from Fermentas were used. PCRs were done with the DreamTaq polymerase from Fermentas. Other DNA modifying enzymes like DNA ligase and PNK were purchased from Roche. Plasmid DNA was extracted using either the Qiagen Plasmid Midi Kit or the Fermentas GeneJET Plasmid Miniprep Kit. For agarose gel purification the Qiagen products QIAquick PCR purification kit or QIAEX II Gel Extraction Kit, in case of fragments smaller than 100 bp, were used.

### Results

### Recombination of random, synthetic attC sites

In order to prove the principle of synthetic attC sites, eight sites were designed that show a random sequence (attCr = random attC). To achieve this goal, an algorithm was generated and used, which generates random sites with the conserved features of attC sites (the steps of the algorithm are summarized in Figure 2).

The four bases (GTTA) at the recombination site were kept and the size of the unpaired central spacer (UCS) was set to five nucleotides. Two extrahelical bases (EHB) were introduced. In the case of the bottom strand, the EHBs are guanine and thymine, five and eleven nucleotides above the UCS in the stem, respectively. Since the variable terminal structure (VTS) decreases the recombination frequency with increasing size, the inventors imposed a small loop of only three base pairs.

The resulting sequences can be seen in a multiple sequence alignment of the eight bottom strands in Figure 3, which underlines the great sequence variety of the sites. Their conserved secondary structures, predicted by the mfold software, are illustrated in Figure 4.

Although all sites have the potential to fold into the ideal structure depicted in Figure 2, the secondary structure predictions show variations. Only two sites, namely attCr 2 and 5, fold correctly. attCr 0, 1, 4, 6 and 7 have a deformation of the UCS, while attCr 0, 1 and 4 only have one correct EHB. The structure of the UTS is changed in the case of attCr 3 and 7 from three to five basepairs. It was therefore expected that the sites recombine with different recombination frequencies owing to their structural variety. The sites 2 and 5 were thought to recombine most efficiently as they showed ideal features. To assess the recombination frequency of the different attC sites, an assay was used that was developed to compare attC x attI recombination by mimicking the cassette integration process.

The so-called suicide conjugation assay is explained in detail above in the materials and method section.

Briefly, the attCr provided by conjugation are carried on a suicide vector from the R6K-based pSW family that depends on the Pir protein (provided by *pir⁺* strains) for its replication. The *pir⁻* recipient strain contains plasmids carrying the integrase and the attI site but does not allow the replication of the suicide pSW-vector. Accordingly, the only way for the suicide plasmid to be maintained in the recipient cell after conjugation, is by integration into the vector carrying the attI site. The recombination can potentially also take place at sites other than attI on the plasmid or in the genome of the recipient cell, but the recombination at these secondary sites is much less efficient than at the attI sites. The attI x attCr recombination leads to the formation of a so-called cointegrate. By selecting for cointegrates, the number of successful recombination events and a corresponding recombination frequency can be deduced.

The results of the suicide conjugation assay with the random attCr sites are shown in Figure 5. The attCr 4 is missing since there were problems with this site during cloning. Though there are variations on the order of four magnitudes in recombination frequency, all random sites were able to recombine with a natural attI site. This outcome confirms that the program generating the random sites was selecting for the right features and it proves the feasibility of the creation of functional synthetic attC sites. Surprisingly, the highest recombination frequency was not obtained by the sites attCr 2 or 5 but by attCr 1, which has deformations in the UCS as well as in the EHB. This finding points out that there are features determining recombination other than those taken into account during the sequence design.

The fact, that almost all sites show a higher recombination frequency than the control is not completely unexpected as the attC_{VCR2/1} is known to recombine with a lower frequency in E. coli than other natural attC sites.

Since the differences in recombination frequency could not be related to the secondary structure in a direct manner, a directed evolution study was conducted to learn more about the factors that influence recombination. For this approach, the site attCr 0 was chosen, as it recombines with an average frequency and a low error rate.

### Directed evolution of random attCr sites

The first step in the directed evolution study was the creation of a library of mutated attCr0 sites, followed by a selection for the mutant with the highest recombination frequency by repeated rounds of the suicide conjugation assay.

Thereby, the attC sites that recombined are retrieved after each conjugation assay and used as the starting point for the next round of selection. Over the different rounds of screening, the population will enrich in highly recombinogenic sites which can be detected by measuring the recombination frequency. When the frequency does not further increase, the maximal frequency has been obtained and the population can be sequenced to elucidate how many different sites sustained and how they differ from the original attCr 0.

### Creation of mutant libraries

The inventors worked with two mutant libraries (L1 and L2) of the attCr 0 that were obtained by two different strategies. L1 was created by using mutagenic PCR, set up in a way that on average, one mutation per attC site was created at random. L2 on the other hand, is based on a more rational approach. Considering the structure of the attCr 0 shown in Figure 6, it is obvious that the mfold predicted unpaired central spacer (UCS) of the site is too large and that this deformation also eliminates the extrahelical G. To elucidate whether the deformed UCS and the absence of the EHB are the reasons for a lower recombination efficiency, L2 contains clones that only vary in eight positions, underlined in Figure 6. This library was obtained by purchasing oligonucleotides that contain the sequence of the attCr 0 but have eight randomized positions (N).

The multiple sequence alignment of the original attCr 0 with 15 or 19 randomly picked clones of L1 and L2, respectively, confirms that the libraries were successfully created (Figure 7). It also reveals that only around 30% of L1 population carried a mutation. Whereas in L2, every clone shows a different sequence and the mutations are mostly restricted to the eight nucleotides that were meant to be mutated.

In order to work with libraries of a comparable population size, the screening was started with around 30,000 clones of L1 and 10,000 clones of L2, which corresponds to approximately 10,000 different attC sites in both libraries.

### Creation of attIr 0

In order to make the directed evolution study work, the inventors ensured that the attC sites can be retrieved from the cointegrates. However, the recombination sites change during the process of attI x attC recombination, as it is illustrated in Figure 8a. Since the strand transfer occurs between G and T of the GTTA sequence, the top of the attI and the attC sites are exchanged in the resulting cointegrate. Thus, a special attI site (attIᵣ 0) had to be designed, whose sequence downstream of the GTTA sequence is identical to the one of the attCᵣ 0. The recombination of the attIᵣ 0 with the attCᵣ 0 can be seen in Figure 8b. The engineered attIᵣ 0 site allows to extract the original attC site after recombination by digestion with ScaI and BamHI.

### Selection process

After establishing both the library of attCr 0 mutants and an attIr 0 site that enables the recovery of the recombined mutants, the screening of the libraries for the mutants with the best recombination properties could be started. The selection is realized by sequential rounds of recombination and recovery of the mutants from the cointegrates as it is explained in Figure 8. Over the different rounds, the libraries will be enriched of mutants that recombine more efficiently than others. This selection process can be followed by the increase of the recombination frequency of the mutant libraries.

### Results of the directed evolution study

The results of the first screening round of both libraries are listed in Table 6. Following one round of selection and with two control *attC* sites; the attC_{VCR2/1} that was also used before and the original attCr 0. As an attI recombination site, the engineered attIᵣ 0 was used for both the libraries and the controls.

**Table 6**

| | **recombination frequency** | **sample size (N)** |
|---|---|---|
| **Libraries** | | |
| L1 | 7.3 8 x 10⁻⁵ ± 3.16 x 10⁻⁵ | 6 |
| L2 | 6:19 x 10⁻⁴ ± 3:39 x 10⁻⁴ | 4 |

| **Positive controls** | | |
|---|---|---|
| attC_{VCR2/1} | 1:92 x 10⁻⁴ ± 9:72 x 10⁻⁵ | 4 |
| attCᵣ 0 | 2:63 x 10⁻⁵ ± 2:13 x 10⁻⁵ | 4 |

The results are surprising, as the recombination frequencies of the controls are lower than those measured in former experiments presented in Figure 5. The inventors therefore directly compared the integration of various attC sites at the natural attI1 site and the modified attIᵣ 0 site. These control experiments revealed that the recombination frequency of attIᵣ 0 is about one order of magnitude smaller than the recombination frequency of attI1 for all attC sites (results not shown).

This result confirms that IntI1 can recombine attC sites of great structural diversity, however IntI1-catalyzed recombination is sensitive to changes in the primary recombination site attI. The fact that the positive control attC_{VCR} increased in recombination frequency compared to former results, cannot be explained and needs to be further investigated. However, the lower recombination efficiency should not affect the selection procedure of the libraries for the mutant possessing the best recombination properties, and apart from the generally lower efficiency, the results are quite expected.

Both libraries show a slight increase in recombination frequency compared to the original attCᵣ 0, reflecting the abundance of mutants with better recombination properties than the original site. It was also expected that the recombination frequency of L2 is higher than that of L1. This can be explained by the fact, that L1 is composed to 60% of the original attCr 0 and only to 30% of mutants,

### attC sites a la carte

A very interesting application of synthetic attC recombination sites is to combine them with functional genetic elements and operators. We thought of two different approaches to show the feasibility and the potential of functional, coding recombination sites: protein linkers and promoters.

In order to combine the sequence requirements of attC sites and of the sequence of choice, i.e. a promoter, a Python script was written that creates a population of random attCr sites and compares them to the sequence of choice by assigning a matching score. Using repeated rounds of mutation, scoring and selection of the attCr sites, the program finally returns sequences that combine both requirements. The assigned score provides information about how well the sequence requirements of the attC sites and the sequence of choice match the output sequence.

### Computing coding attC recombination sites

As an input, the program needs the sequence of choice, i.e. the promoter sequence, and the bases that need to be conserved ("unmutables"). These unmutable residues were shown to be essential to the function of the respective element in previous studies and their mutation will therefore be penalized by the program. As the exact relation between sequence and function is often not completely elucidated, the program is written in a way that it also keeps as many residues as possible of the "mutable" bases of the input sequence. This property increases the chance to obtain a functional site in the end. The information about the sequence requirements of the attC sites, on the other hand, are part of the code and do not need to be provided by the user. Using this stored information, the program creates a population of random attCr sites with the same conserved features as they are presented in Figure 2: the GTTA homology region, an unpaired central spacer of five basepairs (bp) as well as two extrahelical bases. However, the variable terminal sequence (VTS) is not restricted to three residues but can be increased up to 20 bp, as shown in the flowchart in Figure 9.

In the next step the program assigns a score to every attCr site in the population using the following scoring scheme. It iterates over every base of the attCr site and checks if its position is the position of an unmutable in the input sequence. If so, the variable "u" that counts the number of unmutables is raised by one. Subsequently, the program considers the actual bases at that position in the two sequences. If both are the same base, the score for the unmutable bases "sᵤ" is raised by one, if they differ, the score remains unchanged. The same procedure is done in case the considered base of the attCr site is not an unmutable. The only difference here is that the variable "v" is raised by one, counting the number of "mutable bases", and the according score is "sᵥ". Next, the final score of the attCr is calculated. Therefore, the number of conserved residues is normalized by the total number of residues for both, the unmutables ( sᵤ/u ) and mutable residues ( s_{v/}v ). Since the conservation of unmutables is more important than that of other residues, the sᵤ/u is multiplied by ten and the product is added to sᵥ/v resulting in the final score of the attC site. This scoring system rewards both, the conservation of unmutables as well as of all other residues of the input sequence of choice; however, the reward for a conserved unmutable is ten times higher.

The population of attCr sites is then sorted by their score and only the top 50% of the sequences are kept. These sequences are duplicated and each duplicate undergoes a random mutation procedure. Subsequently, the created population is scored again. This cycle of evaluation, selection and mutation is repeated T times (T can be chosen by the user) and in the end, the program returns the highest scoring sequences.

### attC sites coding for inducible promoters

A promoter is a genetic operator needed to initiate gene transcription as they are recognized by transcription factors that in turn recruit RNA polymerase to the transcription start site. Consequently, promoters are found upstream of every open reading frame (ORF).We distinguish between constitutive promoters, which permanently drive gene expression, and inducible promoters, where transcription can be repressed or induced by effector molecules.

Synthetic attC sites coding for promoters could be used to integrate a gene into the bacterial genome or a plasmid and to put it directly under expression of the encoded promoter. We want to design the promoters in a way that they control the expression of the gene cassette located downstream of the recombination site. Transcription can be regulated by the presence of the integrase that catalyzes the attI x attC recombination. If we succeed in creating an attC site that functions as an inducible promoter, gene expression can even be regulated in two ways.

First by the integration of the promoter-attC site at the respective attI target site and secondly by the induction of the promoter by effector molecules. In order to compute attC sites coding for promoters, one has to be aware of the basic architecture of bacterial promoters. Promoters are composed of two hexanucleotides, referred to as -10 and -35 region, according to their position relative to the transcription start site. The hexanucleotides have a conserved sequence as it can be seen in Figure 10A. The spacing between the -10 and -35 regions is in most cases 17 bp (Figure 10 B). Inducible promoters have additional operator sites that are recognized by effector molecules. Usually, the sequence of inducible promoters have two operators that themselves are palindromes [92].

We computed promoter-attC sites for three well studied inducible promoters: PBAD, PLAC and PTET. The procedure that leads to the final sequence is explained on the lac promoter as an example.

The lac promoter is part of the lac operon and can be repressed by the lac repressor as it binds to the promoter region and competes with the RNA polymerase for the access to its binding site. In the presence of lactose, the repressor undergoes a conformational change as it binds to a lactose metabolite called allolactose, which lowers the binding affinity to the DNA operator site and the promoter becomes derepressed [92, 94, 95]. The sequence of a natural lac promoter is depicted Figure 11A [96]. There are two operator sites seen, operator 1 and 2. Natural lac promoters have a third operator (operator 3) site that is not shown here, as it is located 401 bp downstream of the first operator within the open reading frame that is regulated by the lac promoter. It can be seen that the operator site of the natural promoter is not perfectly symmetric. Therefore, Sadler et al. [97] published the construction of a perfectly symmetric lac operator that turned out to have a 10-fold increased repressor binding affinity *in vitro* compared to the wildtype lac operator. Figure 11B compares both the natural and the engineered operator. For the definition of the "unmutables" within the operator, the publication of Lehming et al. [98] was used as they systematically tested the transcriptional repression of mutated, perfectly symmetric lac operators. However, it must be noted that they only introduced mutations in one symmetrical pair in the operator at a time, and that the effect of more than one mutation and the destruction of the perfectly palindromic structure on the transcriptional repression has not been reported. The construction of a synthetic attC site that codes for a lac promoter turned out to be very complicated. The inventors have seen that the secondary structure of the attC site is crucial for its recombination. However, as the operators are palindromic structures, they are prone to form hairpin structures themselves and thereby interfere with the proper folding of the attC site. Furthermore, the fact that the lac promoter is constituted by two repeats of the lac operator that can also interact during secondary structure formation, made it impossible for the program to return a functional consensus sequence.

The only solution to this problem was to only implement one operator site in the attC site. It was shown that by only keeping the lac operator 1, that is closest to the transcription start site, and the operator 3, 400 bp downstream in the ORF, a 700-fold repression is still obtained compared to a 1300-fold repression of the wildtype [99].

The input sequence that was finally used to generate lac promoter-attC sites is depicted in Figure 11C. The wildtype operator sites are changed to the perfectly symmetric sites and the -35 and -10 regions are changed to the consensus sequence of Figure 10A. The position +1 is the start of the ORF and is located directly upstream of the GTTA recombination site of the attC site. This construction allows the expression of a gene directly downstream of the promoter-attC site. For optimal repression of the promoter in absence of lactose, the operator 3 should be implemented in the ORF. The -10 and -35 region together with the operator 1 are defined as unmutables in the program, so that the operator 2 will most probably be mutated in the final consensus sequence generated by the algorithm depicted in Figure 9.

### attC sites as linkers for protein domain shuffling

As indicated besides the engineering of metabolic pathways, synthetic integrons could be used to shuffle protein domains. It is now well established that in several cases, domains can be interchanged to produce new functional proteins. Several recent studies took advantage of the natural modularity of proteins to rewire signaling networks and a family of proteins that could be particularly interesting to engineer with a combinatorial approach include the non-ribosomal polypeptide and polyketide synthetases.

However, changing the domain composition of a protein still involves a great deal of disruption, and testing several designs might be necessary to find working variants. Mutagenesis methods currently used for the production of chimeric protein libraries. In cases where protein modules are well defined and where the presence of a large linker between the domains is not problematic, integrons might be an interesting alternative to existing methods. A synthetic integron enables to generate large number of variants directly in vivo, and a greater diversity of composition than with methods such as SISDC could likely be obtained; with less effort. Furthermore, the orientation of the cassette is preserved and no frameshift can be introduced, which is a great advantage in comparison to methods such as nonhomologous random recombination (NRR). To this end, the attC sites used need to be good protein linkers. In order to make such attC linker, namely that almost only structural features are required for attC recombination, which leaves ample room for modifications of the primary sequence. Mutations are realized so that the structure of the site is preserved, and the sites with the best homology scores to linkers retrieved from a database are selected (LinkerDB: www.ibi.vu.nl/programs/linkerdbwww).

This algorithm allowed the selection of many different *attC* sites. Further work is required to test the functionality of the selected sequences, both as protein linkers and as recombinogenic attC sites. The sequences generated are completely new, and might not all be functional. Experimental measurements will probably reveal new sites with altered functionality. The analysis of such sites will enable to still improve our model of *attC* site folding and recombination. In return, this better knowledge will allow writing a better scoring function for the algorithm, so that new functional sites can be created in ever more efficient ways.

### References

[1] F. de la Cruz et al., Mol Gen Genet., 1988, 25, 211-320.
**[2]** HW. Stokes et al., Molecular microbiology, 1989, 3, 12, 1669-1683.
[3] R. Hall et al., 1998, Drug Resistance Updates, 1, 2, 109-119.
[4] S. Partridge et al., FEMS micro- biology reviews, 2009, 33, 4, 757-784.
[5] D. Mazel et al., Science, 1998, 280, 5363, 605-608.
[6] R. Vaisvila et al., IXth international congress of bacteriology and applied microbiology, 1999.
[7] D. Mazel, Nature reviews. Microbiology, 2006, 4, 8, 608-620.
[8] AM. Carnahan et al., Journal of clinical microbiology, 1994, 32, 7, 1805-1806.
[9] A. Ogawa et al., Microbiology and immunology, 1993, 37, 8, 607-616.
[10] A. Barker et al., Microbiology (Reading, England), 1997, 143 (Pt 6), 1805-1813.
[11] Y. Boucher et al., Trends in microbiology, 2007, 15, 7, 301-309.
[12] DA. Rowe-Magnus et al., Proceedings of the National Academy of Sciences of the United States of America, 2001, 98, 2, 652-657.
[13] G. Cambray et al., Annual review ofgenetics, 2010, 44, 1, 141-166.
[14] CM Collis et al., Molecular microbiology, 1993, 9, 1, 41-52.
[15] CM Collis et al., Antimicrob. Agents Chemother., 1995, 39, 1, 155-162.
[16] T. Jove et al., PLoS genetics, 2010, 6, 1.
[17] C. Levesque et al., Gene, 1994, 142,1, 49-54.
[18] R. Hall et al., Genetica, 1993, 90, 2, 115-132.
[19] GD Recchia et al., Microbiology (Reading, England), 1995, 141 (Pt 12), 3015-3027.
[20] HW Stokes et al., Molecular Microbiology, 1997, 26, 4, 731-745.
[21] GD Recchia et al., Nucleic acids research, 1994, 22, 11, 2071-2078.
[22] C. Collis et al., Molecular Microbiology, 1992, 6, 19, 2875-2885.
[23] S. Nunes-Duby et al., Nucleic Acids Research, 1998, 26, 2, 391-406.
[24] D. Esposito et al., Nucleic acids research, 1997, 25, 18, 3605-3614.
[25] N. Grindley et al., Annual Review of Biochemistry, 2006, 75, 1, 567-605.
[26] D. Gopaul et al., The EMBO Journal, 1998, 17, 14, 4175-4187.
[27] N. Messier et al., Journal of bacteriology, 2001, 183, 22, 6699-6706.
[28] D. MacDonald et al., Nature, 2006, 440, 7088, 1157-1162.
[29] RM Hall et al., Molecular microbiology, 1995, 15, 4, 593-600.
[30] MV Francia et al., Journal of bacteriology, 1996, 178, 3, 894-898.
[31] G. Recchia et al., Molecular Microbiology, 1995, 15, 1, 179-187.
[32] K. Hansson et al., Molecular Microbiology, 1997, 26, 3, 441-453.
[33] V. Francia et al., Molecular Microbiology, 1993, 10, 4, 823-828.
[34] H. Segal et al., FEMS Microbiology Letters, 1997, 153, 2, 321-326.
[35] V. Dubois et al., PloS one, 2007, 2, 12.
[36] E. Guerin et al., Science, 2009, 324, 5930, 1034.
[37] 1. Erill et al., FEMS Microbiology Reviews, 2007, 31, 6, 637-656.
[38] A. Aertsen et al., Trends in microbiology, 2006, 14, 10, 421-423.
[39] W. Kelley, Molecular microbiology, 2006, 62, 5, 1228-1238.
[40] Z. Baharoglu et al., PLoSgenetics, 2010, 6, 10, e1001165.
[41] C. Loot et al., The EMBO Journal, 2010, 29, 15, 2623-2634.
[42] E. Martinez et al., The EMBO Journal, 1990, 9, 4, 1275-1281.
[43] C. Collis et al., Molecular Microbiology, 1998, 29, 2, 477-490.
[44] A. Gravel et al., Nucleic acids research, 1998, 26, 19, 4347-4355.
[45] D.J. Sherratt et al., mobileDNAII, chapter 9"Gene Acquisition in bacteria by integron-mediated site-specific recombination". Number 162-176. Nancy L. Craig, 2002.
[46] D. Rowe-Magnus et al., Genome research, 2003, 13, 3, 428-442.
[47] V. Francia et al., J Bacteriol., 1999, 181, 21, 6844-6849.
[48] C. Johansson et al., Nucleic Acids Research, 2004, 32, 13, 4033-4043.
[49] M. Bouvier et al., The EMBO Journal, 2005, 24, 24, 4356-4367.
[50] M. Bouvier et al., PLoS Genet, 2009, 5, 9, e1000632.
[51] C. Frumerie et al., Nucleic Acids Research, 2009, 38, 2, 559-569.
[52] A. Larouche et al., Mobile DNA, 2011, 2, 1.
[53] AJ. Clark et al., Annual Review of Microbiology, 1962, 16, 1, 289-319.
[54] P. Christie et al., Molecular membrane biology, 2005, 22, 1-2, 51-61.
[55] E. Grohmann et al., Microbiology and molecular biology reviews : MMBR, 2003, 67,2.
[56] TD Lawley et al., FEMS Microbiology Letters, 2003, 224, 1, 1-15.
[57] J. W. Foster et al., Microbiology: An Evolving Science, 2009, W. W. Norton and Company Ltd..
[58] M. Llosa et al., Molecular Microbiology, 2002, 45, 1, 1-8.
[59] C. Alvarez-Martinez et al., Microbiology and molecular biology reviews : MMBR, 2009, 73, 4, 775-808*.*
[60] W. Pansegrau et al., Proceedings of the National Academy of Sciences of the United States of America, 1993, 90, 7, 2925-2929.
[61] W. Pansegrau et al., Journal of Biological Chemistry, 1996, 271, 22, 13068-13076.
[62] C. Elvira Cesar et al., Molecular microbiology, 2006, 62, 4, 984-996.
[63] O. Draper et al., Proceedings of the National Academy of Sciences of the United States of America, 2005, 102, 45, 16385-16390.
[64] P. Garcillan-Barcia et al., Molecular microbiology, 2007, 63, 2, 404-416.
[65] D. Endy, Nature, 2005, 438, 7067, 449-453.
[66] Syntheticbiology. org, August 2011.
[67] N. Rosenfeld et al., Molecular Systems Biology, 2007, 3.
[68] S. Basu et al., Nature, 2005, 434, 7037, 1130-1134.
[69] V. de Lorenzo et al., EMBO reports, 2008, 9, 9, 822-827.
[70] M. Schmidt, Synthetic Biology: The Technoscience and Its Societal Consequences. Springer, 2009.
[71] S. Leduc. La biologie synthétique, étude de biophysique. Poinat, 1912.
[72] international, genetically engineered machines: www.igem.org, 2011.
[73] Registry of standard biological parts: partsregistry.org, 2011.
[74] T. Ellis et al., Nature Biotechnology, 2009, 27, 5, 465-471.
[75] RC Cadwell et al., PCR methods and applications, 1992, 2, 1, 28-33.
[76] E. Haseltine et al., Annual review of biophysics and biomolecular structure, 2007, 36, 1, 1-19.
[77] J. Kolkman et al., Nature Biotechnology, 2001, 19, 5, 423-428.
[78] Y. Yokobayashi et al., Proceedings of the National Academy of Sciences of the United States of America, 2002, 99, 26, 16587-16591.
[79] J. Brannigan et al., Nature Reviews Molecular Cell Biology, 2002, 3, 12, 964-970.
[80] C. Tuerk et al., Science (New York, N.Y.), 1990, 249, 4968, 505-510.
[81] RD Jenison et al., Science, 1994, 263, 5152, 1425-1429.
[82] M. Wieland et al., Angewandte Chemie International Edition, 2008, 47, 14, 2604-2607.
[83] O Rackham et al., Biochemical Society transactions, 2006, 34(Pt 2), 328-329.
[84] A. Crameri et al., Nat Biotech, 1997, 15, 5, 436-438.
[85] D. Bikard, Study of the integron recombination mechanism, and integron use as a genetic shuffling device for biotechnological purpose. PhD thesis, Universite Paris Diderot (Paris 7), 2010.
[86] D. Bikard et al., Nucleic Acids Research, 2010, 38, 15, e153-e153.
[87] L. Biskri et al., J Bacteriol., 2005, 187, 5, 1740-1750.
[88] G. Demarre et al., Nucleic Acids Research, 2007, 35, 19, 6475-6489.
[89] R. Grunberg et al., Nucleic acids research, 2010, 38, 8, 2663-2675.
[90] Protein linker database: http://www.ibi.vu.nl/programs/linkerdbwww/, 2011.
[91] R. George et al., Protein engineering, 2002, 15, 11, 871-879.
[92] F. Jacob et al., Journal of molecular biology, 1961, 3, 318-356.
[93] Alberts et al., Garlandscience, 5th edition, 2008.
[94] Gilbert et al.. Protein-ligand interaction, pages 193-210. de Gruyter, Berlin, 1975.
[95] D. Galas et al., Nucleic Acids Research, 1978, 5, 9, 3157-3170.
[96] R. Lutz et al., Nucleic acids research, 1997, 25, 6, 1203-1210.
[97] JR. Sadler et al., Proceedings of the National Academy of Sciences, 1983, 80, 22, 6785-6789.
[98] N. Lehming et al., The EMBO Journal, 1987, 6, 10, 3145-3153.
[99] S. Oehler et al., The EMBO Journal, 1990, 9, 4, 973-979.
[100] L. Ferrieres et al., Journal of bacteriology, 2010, 192, 24, 6418-6427.
[101] M. Herrero et al., J. Bacteriol., 1990, 172, 11, 6557-6567.
[102] J. Sambrook et al. Molecular cloning: a laboratory manual, volume 3. Cold Spring Harbor Laboratory Press, 2001.
[103] G. Demarre et al., Research in Microbiology, 2004, 156, 2, 245-255.
[104] C. Walsh, Science, 2004, 303, 5665, 1805-1810.
[105] C. Khosla et al., Current opinion in chemical biology, 2009, 13, 2, 135-143.
[106] Z. Dosztányi et al., J Mol. Biol., 2005, 347, 827-839.
[107] Z. Dosztányi et al., Bioinformatics, 2005 21, 3433-3434.

## Claims

1. An isolated DNA molecule consisting of a sequence comprising at least the elements:
N₁-N₂-N₃-N₄-N₅-N₆-N₇-N₈-N₉ (SEQ ID NO: 1) in the order specified;
wherein N₁ comprises at least 4 nucleotides of which the third from last nucleotide is A and N₁ is complementary to N₉, N₂ if present comprises no more than 2 nucleotides, N₃ comprises 6 to 10 nucleotides and is non-complementary to N₇; N₄ comprises 9 to 30 nucleotides and is complementary to N₆; N₅ comprises 3 to 50 nucleotides; N₆ comprises 9 to 30 nucleotides and is complementary to N₄ which comprises at least one nucleotide which forms a extra helical base pair when N₄ and N₆ form a double helical strand; N₇ comprises 6 to 10 nucleotides and is non-complementary to N₃; N₈ if present comprises no more than 2 nucleotides; N₉ comprises at least 4 nucleotides of which the second from last nucleotide is T and N₉ is complementary to N₁; and wherein said isolated DNA molecule forms a single stranded cruciform structure.

2. An isolated DNA molecule according to claim 1 in which portions N₃ & N₇ comprise the same number of nucleotides.

3. An isolated DNA molecule according to claim 1 in which portions N₃ & N₇ comprise a different number of nucleotides.

4. An isolated DNA molecule according to any one of claims 1 to 3, in which portion N₆ comprises at least one additional EHB.

5. An isolated DNA molecule according to any one of claims 1 to 4, in which said isolated DNA molecule encodes transcription promoter or a protein binding site or an in-frame protein linker.

6. A vector comprising a sequence consisting of TAACN₃N₄N₅N₆N₇GTTA (SEQ ID NO: 1).

7. A vector according to claim 7 further comprising a multiple cloning site (MCS).

8. A method to generate a *de novo* attC site comprising the steps:
a) generating a mixed population of putative attCr sites each of which comprises a sequence N₁-N₂-N₃-N₄-N₅-N₆-N₇-N₈-N₉ (SEQ ID NO: 1);
b) selecting a sequence to which the finalized attC site should be as identical to as possible, specifying in the selected sequence those nucleotides must be conserved called 'u' positions and those that do not need to be conserved called 'v' positions;
c) aligning as best as possible each attCr site from step a) with the selected sequence;
d) the attCr sites in the population are scored using a scoring scheme based upon comparing each 'u' nucleotide present in each position of the attCr and selected sequence, wherein if both are the same base, the score for the attCr site is raised by one and if they differ the score remains unchanged, the cumulative total being Sᵤ; and comparing each 'v' nucleotide present in each position of the attCr and selected sequence, wherein if both are the same base, the score for the attCr site, the cumulative total being Sᵥ;
e) a final score for each attCr site in the library is calculated as the sum of the ratio of Sᵤ to the total number of 'u' nucleotides and the ratio of Sᵥ to the total number of 'v' nucleotides, wherein the ratio of Sᵤ to the total number of 'u' nucleotides is multiplied by a factor of 10;
f) the population of attCr sites is sorted by their final score and the top 50% of the attCr sites are retained;
g) the population of attCr sites from step f) are duplicated and each duplicate undergoes a random mutation procedure and each member of this population is scored against the selected sequence
h) steps of scoring, selection and further mutation (steps a) to g)) are repeated until no further increase in the highest scoring attCr sites occurs.

9. A method to test the properties of a *de novo* attC site, comprising the steps of:
a) preparing a conjugation competent donor strain of bacteria a first plasmid comprising the attC site to be tested said plasmid also comprising a first resistance marker in functional proximity to said attC site, wherein said first plasmid is suitable for conjugational transfer and wherein said donor strain is an auxotroph;
b) preparing a recipient strain comprising a second plasmid capable of expressing the ORF of the intl integrase and a second resistance marker, and a third plasmid containing the attI recombination which also comprises a third resistance marker;
c) mixing the donor and recipient strains under conditions wherein conjugation occurs between them;
d) growing equal portions of the resulting mixed population under selection conditions designed to eliminate the auxotrophic donor strain and to test for either the presence of the first selection marker or the second selection marker;
e) comparing the ratio of the populations showing resistance to the first selection marker versus those showing resistance to the second selection marker so as to calculate the recombination frequency of the tested attC site.
